# EUROPÄISCHE PATENTANMELDUNG

(11) **EP 1 750 128 A2**
(43) Veröffentlichungstag der Anmeldung: **07.02.2007**
(21) Anmeldenummer: 06015526.4
(22) Anmeldetag: 26.07.2006
(51) Int. Cl.: G01N 33/44, G01N 21/31, G01N 29/024

(54) **Verfahren und Vorrichtung zur Ermittlung des Reifezustandes von Duroplast-Zusammensetzungen**

(30) Priorität: 04.08.2005 DE 102005036643
(71) Anmelder: Fraunhofer-Gesellschaft zur Förderung der angewandten Forschung e.V., 80686 München (DE)
(72) Erfinder: Götz, Joachim, 83064 Raubling (DE); Bräuning, Rüdiger, 76703 Kraichtal (DE); Becker, Wolfgang, 76131 Karlsruhe (DE); Henning, Frank, 76327 Pfinztal (DE)
(74) Vertreter: Lenz, Steffen

(57) **Zusammenfassung**

Es wird ein Verfahren zur Ermittlung des Reifezustandes, insbesondere der Viskosität, von Duroplast-Zusammensetzungen vorgeschlagen. Zur Qualitäts- und Prozeßkontrolle in Echtzeit ist dabei vorgesehen, daß die Viskosität einer jeweiligen Duroplast-Zusammensetzung zerstörungsfrei ermittelt wird, indem zunächst zu verschiedenen Zeitpunkten während des Reifens der Duroplast-Zusammensetzung die Viskosität wenigstens einer Probe dieser Zusammensetzung mittels herkömmlicher, nicht zerstörungsfreier Verfahren zur Ermittlung der Viskosität bestimmt wird, wobei die Duroplast-Zusammensetzung im wesentlichen zu denselben Zeitpunkten mittels spektroskopischer Verfahren oder Ultraschall untersucht wird, wonach aus den erhaltenen Meßwerten eine Korrelation der mittels der spektroskopischen Verfahren oder Ultraschall erhaltenen Meßwerte mit den mittels der herkömmlichen Verfahren erhaltenen Viskositätswerte erstellt wird. Anschließend wird dann die Viskosität dieser Duroplast-Zusammensetzung ausschließlich mittels der spektroskopischen Verfahren oder Ultraschall unter Berücksichtigung der erhaltenen Korrelation ermittelt. Die Erfindung betrifft ferner eine zur Durchführung eines solchen Verfahrens geeignete Vorrichtung zur Herstellung duroplastischer Halbzeuge.

## Beschreibung

Die Erfindung betrifft ein Verfahren zur Ermittlung des Reifezustandes, insbesondere der Viskosität, von Duroplast-Zusammensetzungen. Sie ist ferner auf eine Vorrichtung zur Herstellung duroplastischer Halbzeuge mit einer Mischeinrichtung zur Homogenisierung der eingesetzten Komponenten und einer Reifungseinheit, insbesondere in Form einer Bestrahlungs- und/oder Scherungseinheit, gerichtet.

Duroplastische Polymere finden aufgrund ihrer günstigen mechanischen Eigenschaften verbreitet industriellen Einsatz, beispielsweise als Formteile für die Fahrzeugindustrie (z.B. für Motorhauben, Heckklappen, Verkleidungsteile, Stoßfänger, Windabweiser etc.), für die Telekommunikations- und Elektrik- bzw. Elektronikindustrie (z.B. für Gehäuse, wie Schaltkästen usw., Mobilfunkantennenanlagen etc.) und dergleichen mehr. Zur Erhöhung ihrer Schlagzähigkeit kommen häufig faserverstärkte Duroplaste zum Einsatz.

Im Gegensatz zu thermoplastischen Polymeren, welche durch Erwärmen ab einer bestimmten Temperatur plastisch verformbar sind, handelt es sich bei Duroplasten um Werkstoffe, bei welchen in der Regel flüssige oder viskose Harze mit geringer Molmasse zu dem festen, vernetzten Duroplast mit demgegenüber erheblich größerer Molmasse chemisch/physikalisch ausgehärtet bzw. vernetzt werden. Dabei werden die eingesetzten Harzkomponenten gemeinsam mit gegebenenfalls erwünschten Additiven, wie Katalysatoren, Härtern (zumeist peroxidischen Härtern), Eindickungsmitteln (z.B. Magnesiumoxid, MgO), Antioxidantien, Flammschutzmitteln, Trennmitteln (z.B. Zinkstearat), Inhibitoren, Lösungsmitteln (z.B. Styrol), Farbstoffen bzw. Pigmenten, Stabilisatoren, Dispergierungsmitteln usw., Füllstoffen (z.B. Kreide) und Verstärkungfasern (z.B. Glas-, Carbon-, Aramidfasern, natürlichen Verstärkungsfasern etc.) gemischt und zu Halbzeugen verarbeitet.

Die genannten duroplastischen Halbzeuge kommen gegenwärtig vornehmlich in Form von SMC-Formmassen (Sheet Molding Compounds), BMC-Formmassen (Bulk Molding Compounds) oder auch rieselfähigen Duroplasten zum Einsatz, wobei deren weltweite Produktion im Jahr 1996 1,1 Millionen Tonnen betrug. Während die SMC- und BMC-Formmassen mehr oder minder teigartig viskose duroplastische Massen darstellen, bei welchen die eingesetzten Komponenten bereits vollständig gemischt und zur Endverarbeitung bereit vorliegen, handelt es sich bei den rieselfähigen Duroplasten um im wesentlichen feste Granulate, deren Vernetzungsgrad jedoch noch hinreichend gering ist, um sie unter Einwirkung von Temperatur und/oder Druck zu plastifizieren und sie so gänzlich auszuhärten bzw. zu vernetzen, so daß sie nicht mehr plastifizierbar sind. Lediglich exemplarisch für eine verbreitet eingesetzte Duroplast-Zusammensetzung seien ungesättigte Polyesterharze genannt, welche insbesondere unter Zugabe von Füllstoffen, Fasern und Additiven mittels Fließpressen (Compression Molding), Spritzgießverfahren (Injection Molding), Harzinfusionsverfahren (Resin Transfer Molding), Extrusionsverfahren (Pultrusion) oder dergleichen verarbeitet werden.

Bei der Herstellung solcher SMC-Formmassen durch das sogenannten Fließpreßverfahren (Compression Molding), werden Lang- bzw. Endlosfasern (z.B. Glasfasern), sogenannte Rovings, von Spulen abgezogen und mittels einer Schneideinrichtung auf die gewünschte Länge zugeschnitten. Diese geschnittenen Fasern werden dann auf eine Trägerfolie überführt, auf welche ein Anteil, z.B. etwa die Hälfte, der erforderlichen Menge der eingesetzten Duroplast-Zusammensetzung, in der Regel ein Harz-/Füllstoffgemisch, in Form eines dünnen Filmes aufgetragen worden ist. Nach dem Abdekken dieser Trägerfolie mittels einer weiteren Trägerfolie, auf welche der restliche Anteil der eingesetzten Duroplast-Zusammensetzung aufgetragen worden ist, durchläuft diese sandwichartige Anordnung eine Walzenstrecke, in welcher eine Imprägnierung der Fasern und eine Verdichtung des zu erhaltenden Halbzeugs erreicht wird. Das z.B. mehrere Millimeter dicke, flächenförmige Halbzeug wird schließlich entweder aufgewickelt oder zu Zuschnitten in der gewünschten Größe konfektioniert.

Im Falle der BMC-Formmassen, welche auch als "Stopfmassen" bezeichnet werden, wird die eingesetzte Duroplast-Zusammensetzung über Schneckenspritzgießmaschinen verarbeitet.

Die DE 102 11 920 A1 beschreibt ein weiteres Verfahren zur Herstellung von duroplastischen Halbzeugen, indem die eingesetzte Duroplast-Zusammensetzung zunächst in einer Mischeinrichtung, insbesondere in Form eines ersten Extruders, Rührers oder Kneters, gemischt und sodann die Verstärkungsfasern zugesetzt werden, was vorzugsweise in einem zweiten Extruder geschehen kann, in welchen die homogenisierte Duroplast-Zusammensetzung überführt worden ist.

In jedem Fall - sei es bei SMC- oder BMC-Formmassen oder bei mittels eines Verfahrens gemäß der DE 102 11 920 A1 hergestellten Duroplast-Halbzeugen - muß im Anschluß an die Mischung der eingesetzten Komponenten eine Reifung der Duroplast-Zusammensetzung erfolgen, um die Viskosität der fertig gemischten Duroplast-Zusammensetzung zu erhöhen. Während für den Mischprozeß eine möglichst geringe Viskosität erwünscht ist, um eine optimale Homogenisierung sowie eine einwandfreie Imprägnierung der Fasern zu erreichen, ist zur Weiterverarbeitung bzw. Lagerung der Duroplast-Halbzeuge eine demgegenüber deutlich höhere Viskosität erforderlich, um insbesondere einem Entmischen der Komponenten vorzubeugen und eine zuverlässige Handhabung (beim Transport, Zuschneiden, Stapeln etc. sowie bei der Endverarbeitung, anläßlich welcher eine praktisch vollständige Vernetzung zu dem jeweiligen Duroplast-Formteil erfolgt) zu garantieren. Desgleichen erfordert eine hochwertige Oberflächenqualität des fertigen Formteils eine gewisse Viskosität der Duroplast-Zusammensetzung.

Während der Reifung findet eine gewisse teilweise Vernetzung der eingesetzten Harze statt, wodurch die Viskosität der Zusammensetzung erhöht wird, die Formmasse aber gleichwohl plastisch verformbar bleibt, um sie anläßlich der Endverarbeitung in die gewünschte Form zu bringen und gänzlich auszuhärten. Eine Reifung der Duroplast-Zusammensetzung kann entweder durch reine Lagerung über mehrere Tage bis Wochen oder durch Energiezufuhr, z.B. durch Erwärmen, mechanische Beanspruchung (insbesondere Scherung) oder durch Bestrahlen (z.B. mit elektromagnetischen Wellen, insbesondere UV-Strahlen oder Mikrowellen, mit α- oder β-Strahlung etc.) geschehen, wie es beispielsweise gemäß der DE 102 11 920 A1 vorgesehen ist. Desgleichen kann der Reifungsprozeß durch eine geeignete Rezeptur der Zusammensetzung (z.B. durch das jeweilige Harzsystem, durch Kombination reaktiver Systeme etc.) beschleunigt werden. Der Reifungsprozeß läßt sich auf diese Weise auf eine Dauer von gegebenenfalls einigen Minuten verkürzen.

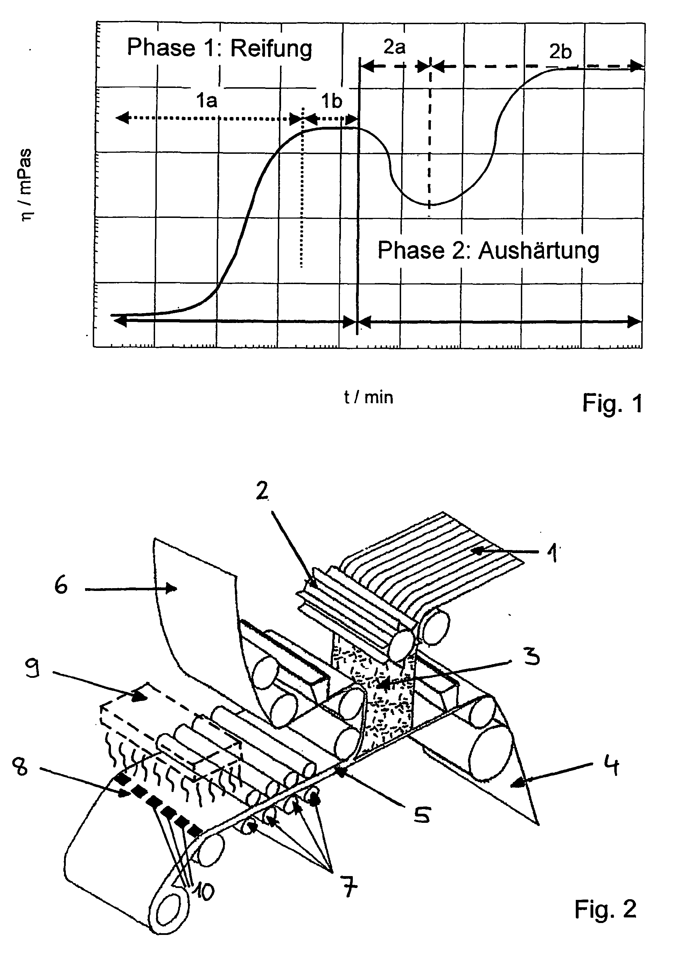

In Fig. 1 ist ein typisches Reifungsverhalten einschließlich des nachfolgenden Aushärtens anläßlich der Endverarbeitung zum Formteil einer Duroplast-Zusammensetzung dargestellt, wobei die Viskosität η der Duroplast-Zusammensetzung als Funktion der Zeit t in doppelt logarithmischer Skalierung wiedergegeben ist. Die Reifung geschieht während "Phase 1", während die anschließende Aushärtung ("Curing" bzw. "Molding") während "Phase 2", z.B. in einem Preßwerkzeug, erfolgt. Die Reifung ("Phase 1") umfaßt beispielsweise die Lagerzeit unmittelbar nach Mischen der die Duroplast-Zusammensetzung bildenden Komponenten und wird auch als "Eindickung", "Thickening" oder "Maturation" bezeichnet. Sie kann in eine "Phase 1a" der Eindickung und in eine hieran zeitlich anschließende "Phase 1b" der Lagerung ("Aging") aufgeteilt werden. Wie aus Fig. 1 ersichtlich, finden während der Reifung gewisse chemische Vernetzungsvorgänge statt, welche in der Regel zu einer räumlich begrenzten Netzwerkbildung führen, so daß die mittlere Molmasse zunimmt und die Viskosität um bis zu einige Größenordnungen, beispielsweise von etwa 20 Pas bis etwa 50.000 Pas, ansteigt. Die Aushärtung ("Phase 2"), welche zu einem praktisch nicht mehr verformbaren, vernetzten Formteil führt, läßt sich ebenfalls in zwei Phasen unterteilen, nämlich in eine "Phase 2a", innerhalb welcher die Viskosität der Duroplast-Zusammensetzung infolge der Erwärmung kurzzeitig wieder abnimmt, und in eine "Phase 2b", innerhalb welcher die Vernetzungsreaktionen zu der duroplastischen Polymermatrix stattfinden. Im Rahmen dieser Ausbildung eines dreidimensionalen Netzwerkes nimmt die mittlere Molmasse und folglich die Viskosität stark zu.

Problematisch ist stets, daß der Reifungsvorgang nur bedingt überwacht bzw. nur bedingt eingeschätzt werden kann, wann die Duroplast-Zusammensetzung den gewünschten Reifegrad bzw. die gewünschte Viskosität erreicht hat. Zur Ermittlung des Reifungszustandes von Duroplast-Zusammensetzungen werden herkömmlich bekannte Verfahren zur Ermittlung der Viskosität herangezogen, wobei der Zusammensetzung Proben entnommen werden, welche unmittelbar anschließend mittels Viskosimetern, wie Rotationsviskosimetern (z.B. Brookfield™), Rheometern (z.B. Platte/Platte oder Platte/Kegel), in oszillatorischer oder translatorischer Rheometrie und dergleichen rheometrisch untersucht werden. Nachteilig ist insbesondere, daß auf diese Weise keine zerstörungsfreie Ermittlung des Reifungszustandes bzw. der Viskosität der Duroplast-Zusammensetzung möglich ist und daß andererseits praktisch keine Echtzeit- bzw. Online-Messungen durchgeführt werden können, wie sie insbesondere im Falle einer mittels Energieeintrag beschleunigten Reifung, aber auch einer einfachen Lagerung wünschenswert wären. Hinzukommt, daß die Bereitstellung von duroplastischen Halbzeugen mit reproduzierbarer und gleichbleibender Qualität ohnehin aufgrund der Reaktionsfreudigkeit der noch nicht gänzlich ausgehärteten Harze nur sehr schwer gewährleistet werden kann, da die Reifung stark von der Umgebungstemperatur, (Luft)feuchtigkeit und anderen äußeren Einflüssen abhängig ist, wodurch es zu einem nicht unerheblichen Ausschuß kommen oder zumindest eine Nachbearbeitung des fertigen Formteils erforderlich sein kann. Auch insoweit besteht folglich ein Bedarf an einer einfachen und kostengünstigen, zerstörungsfreien Echtzeitmessung des Reifungszustandes bzw. der Viskosität der Duroplast-Zusammensetzung.

Der Erfindung liegt die Aufgabe zugrunde, hier Abhilfe zu schaffen.

Erfindungsgemäß wird diese Aufgabe bei einem Verfahren zur Ermittlung des Reifezustandes, insbesondere der Viskosität, von Duroplast-Zusammensetzungen dadurch gelöst, daß die Viskosität einer jeweiligen Duroplast-Zusammensetzung zerstörungsfrei ermittelt wird, indem zunächst zu verschiedenen Zeitpunkten während des Reifens Proben der Duroplast-Zusammensetzung die Viskosität wenigstens einer Probe dieser Zusammensetzung mittels herkömmlicher, nicht zerstörungsfreier Verfahren zur Ermittlung der Viskosität bestimmt wird, wobei die Duroplast-Zusammensetzung im wesentlichen zu denselben Zeitpunkten mittels spektroskopischer Verfahren oder Ultraschall untersucht wird, wonach aus den erhaltenen Meßwerten eine Korrelation der mittels der spektroskopischen Verfahren oder Ultraschall erhaltenen Meßwerte mit den mittels der herkömmlichen Verfahren erhaltenen Viskositätswerte erstellt wird, und indem anschließend die Viskosität dieser Duroplast-Zusammensetzung ausschließlich mittels der spektroskopischen Verfahren oder Ultraschall unter Berücksichtigung der erhaltenen Korrelation ermittelt wird.

Zur Lösung dieser Aufgabe sieht die Erfindung ferner bei einer Vorrichtung zur Herstellung duroplastischer Halbzeuge mit einer Mischeinrichtung zur Homogenisierung der eingesetzten Komponenten und einer Reifungseinheit, insbesondere in Form einer Bestrahlungs- oder Scherungseinheit, vor, daß der Reifungseinheit wenigstens eine zerstörungsfreie Meßeinheit in Form einer elektromagnetischen Strahlungsquelle sowie eines zugehörigen Sensors zum Erfassen der jeweiligen elektromagnetischen Strahlungsspektren, wenigstens eines Elektromagnets sowie eines NMR-Sensors zum Erfassen der jeweiligen Kernresonanzspektren und/oder wenigstens eines Ultraschallsenders sowie eines Ultraschallmikrofons zum Erfassen der jeweiligen Ultraschallsignale zugeordnet ist, wobei der jeweilige Sensor und/oder das Ultraschallmikrofon mit einer Auswerteeinheit korrespondiert.

Die Erfindung ermöglicht auf einfache Weise eine zerstörungsfreie Echtzeitmessung der Viskosität der reifenden Duroplast-Zusammensetzung, wobei gefunden wurde, daß die jeweils mittels spektroskopischer Verfahren erhaltenen Spektren oder mittels Messung z.B. der Geschwindigkeit und/oder Dämpfung von in die Zusammensetzung eingekoppeltem Ultraschall erhaltenen Meßwerte repräsentativ für die jeweilige Viskosität einer bestimmten Zusammensetzung sind. Es muß somit lediglich für eine gegebene Zusammensetzung eine Korrelation, d.h. ein funktionaler Zusammenhang anhand einer Eich- bzw. Kalibrierkurve, der mittels der spektroskopischen Verfahren oder Ultraschall erhaltenen Meßwerte mit den mittels der herkömmlichen Verfahren erhaltenen Viskositätswerte erstellt werden. Auf diese Weise wird - in der Regel mittels einer Datenverarbeitungseinheit - aus dem mittels der herkömmlichen, direkten, nicht zerstörungsfreien Verfahren zur Ermittlung der Viskosität durch Probennahme erhaltenen Meßdatensatz sowie dem etwa zeitgleich bzw. zu etwa denselben Zeiten (Reifungszeiten) an gleichen Proben durch die indirekten, zerstörungsfreien Verfahren erhaltenen, abgeleitenen Datensatz eine für ein und dieselbe Zusammensetzung spezifische Korrelation erhalten, welche anschließend eine einfache und kostengünstige Echtzeitmessung der Viskosität allein auf der Basis der genannten, zerstörungsfreien Meßverfahren (Spektroskopie oder Ultraschall) gewährleistet. Während der Duroplast-Zusammensetzung zur Ermittlung der Viskosität mittels der herkömmlichen, nicht zerstörungsfreien Verfahren für die Erstellung der Korrelation selbstverständlich auch mehrere Proben zu verschiedenen Zeitpunkten entnommen werden können, kann es insbesondere zweckmäßig sein, der frischen Duroplast-Zusammensetzung nur anfänglich eine oder mehrere Proben zu entnehmen und diese Probe(n) zu verschiedenen Zeitpunkten mittels der nicht zerstörungsfreien Verfahren zu untersuchen, während die eigentliche Zusammensetzung etwa zeitlich parallel mittels der spektroskopischen Verfahren oder Ultraschall untersucht wird.

Ferner lassen sich aus den mittels der zerstörungsfreien Meßverfahren erhaltenen Werte unter Einsatz von chemometrischen Methoden (multivariate Datenanalyse, MVDA) auch andere Parameter, wie das Fließverhalten, der E- oder Schubmodul, die Partikelgröße(nverteilung) von Füllstoffen/Fasern, die Molmasse(nverteilung) der sich teilweise vernetzenden Harzkomponenten, der Vernetzungsgrad, die Morphologie, der Feuchte- oder Wassergehalt, welcher bei der Vernetzung entsteht, etc., ableiten. In den meisten Fällen ist der Einsatz einer geeigneten Signalverarbeitung der mittels der zerstörungsfreien Verfahren erhaltenen Meßwerte, wie Filteroperationen zum Glätten, Kontrasterhöhungen und dergleichen, zweckmäßig, um den Grad der Korrelation zwischen den mittels der herkömmlichen, nicht zerstörungsfreien und der zerstörungsfreien Verfahren erhaltenen Datensätzen zu erhöhen. Schließlich lassen sich aus den Datensätzen auch numerische Modelle ableiten, welche eine Vorhersage des Reifezustandes einer jeweiligen Duroplast-Zusammensetzung erlauben. Das erfindungsgemäße Verfahren ermöglicht folglich eine optimale zerstörungsfreie Prozeß- und Qualitätskontrolle der reifenden Duroplast-Zusammensetzung in Echtzeit unter Vermeidung von zeit- und kostenintensiven Probennahmen und ohne Unterbrechung bzw. Beeinflussung des Herstellungs- bzw. Reifungsprozesses von Halbzeugen aus Duroplast-Zusammensetzungen. Dabei kann insbesondere der optimale Zeitpunkt zur Endverarbeitung (Aushärtung), d.h. das Ende der "Phase 1" gemäß Fig. 1, ermittelt werden, wodurch einerseits ein zu frühes Endverarbeiten einer noch nicht hinreichend gereiften Duroplast-Zusammensetzung, andererseits zu lange Lager- bzw. Reifungszeiten vermieden werden können. Das erfindungsgemäße Verfahren kann im Rahmen beliebiger Produktionsschritte, z.B. bereits während der Dosierung und Mischung der Duroplast-Zusammensetzung (z.B. in Extrudern oder Mischern), während der gegebenenfalls durch Energieeintrag unterstützten Reifung, der Portionierung (z.B. mittels Schneideinrichtungen, Bandförderern, Robotern etc.) bis hin zur Endverarbeitung zum fertigen Formteil eingesetzt werden.

Ein weiterer Vorteil des erfindungsgemäßen Verfahrens besteht schließlich darin, daß eine problemlose Untersuchung von auch in hohem Maß mit Verstärkungsfasern versetzten Duroplast-Zusammensetzungen möglich ist, welche bei herkömmlichen, nicht zerstörungsfreien - rheologischen - Verfahren (z.B. Brookfield™) zur Ermittlung der Viskosität eine erhebliche Erhöhung der Reibung zwischen den relativ zueinander bewegbaren Teilen der Meßapparatur bewirken können, so daß es zu Meßfehlern (nicht viskometrische Strömung, Bildung von Hohlräumen, Ablösen des Probematerials von der Rührergeometrie, Verdunsten von insbesondere leicht flüchtigen Bestandteilen der Duroplast-Zusammensetzung, beispielsweise Styrol, etc.) kommen kann. Rheologische Standardverfahren, wie oszillatorische Schermessungen, erlauben hingegen nur Echtzeit- bzw. Online-Messungen an einer einzigen Probe nach der Probenahme, während Messungen während eines jeweiligen Produktionsschrittes, wie sie wünschenswert sind und erfindungsgemäß gewährleistet werden, jedenfalls nicht möglich sind.

Was die erfindungsgemäße Vorrichtung betrifft, so können die jeweils erforderlichen Sensoren/Mikrofone unmittelbar an oder mit Abstand zu der reifenden Duroplast-Zusammensetzung angeordnet sein, wobei die Meßdaten je nach gewähltem zerstörungsfreien Meßverfahren z.B. über Lichtfasern oder elektrische Leitungen an das Spektrometer bzw. die Auswerteeinheit übertragen werden können. Die Auswerteeinheit kann mit mehreren Sensoren/Mikrofonen, also mit mehreren Meßstellen, korrespondieren, so daß mehrere örtliche Meßpunkte der Duroplast-Zusammensetzung gleichzeitig oder in definierten Zeitabständen nacheinander erfaßt werden können.

Selbstverständlich kann das erfindungsgemäße Verfahren jedoch nicht notwendigerweise nur in Verbindung mit einer solchen Vorrichtung zur Herstellung duroplastischer Halbzeuge eingesetzt werden, sondern es sind auch mit geeigneten Sensoren/Mikrofonen ausgestattete Handmeßgeräte, Probenwechsler, Roboter oder dergleichen denkbar, mittels welchen die Viskosität bzw. der Reifungszustand einer jeweiligen Duroplast-Zusammensetzung zerstörungsfrei bestimmt werden kann.

Eine bevorzugte Ausführungsform des erfindungsgemäßen Verfahrens sieht vor, daß ein spektroskopisches Verfahren auf der Basis von elektromagnetischer Strahlung eingesetzt wird.

Hierbei kommt vorzugsweise ein spektroskopisches Verfahren auf der Basis von Infrarot-Strahlung, insbesondere Nahe-Infrarot-Strahlung (NIR mit einer Wellenlänge von etwa 760 nm bis etwa 2,5 µm), mittlere Infrarot-Strahlung (MIR mit einer Wellenlänge von etwa 2,5 bis etwa 25 µm) und/oder ferne Infrarot-Strahlung (FIR mit einer Wellenlänge von etwa 25 µm bis etwa 100 µm), in Betracht. Die IR-Spektroskopie beruht auf der Tatsache, daß die Energie vieler Molekülschwingungen im infraroten Bereich des elektromagnetischen Wellenspektrums liegt. Folglich kann aufgrund der Absorptionsbanden in IR-Spektren auf vorhandene Moleküle oder Molekülgruppen geschlossen werden, welche wiederum repräsentativ für die Viskosität der reifenden Duroplast-Zusammensetzung sind und - nach der vorerwähnten Korrelation mit den direkt bzw. nicht zerstörungsfrei ermittelten Viskositätswerten - eine unmittelbare Ermittlung der Viskosität bzw. des Reifungszustandes der Duroplast-Zusammensetzung erlauben. Die Lage und die Intensität der Absorptionsbanden sind grundsätzlich stoffspezifisch, wobei durch Vergleich der Spektren bekannter Substanzen mit Spektren unbekannter Substanzen letzte identifiziert werden können. Folglich erhält man durch das IR-Spektrum auch Hinweise auf den molekularen Aufbau von unbekannten Verbindungen, welche sich während der Reifung, insbesondere durch die teilweise chemische Vernetzung der eingesetzten Harzkomponenten, bilden. Auf der Grundlage des Lambert-Beer'schen Gesetzes oder durch Kalibrierung/Eichung sind nicht nur qualitative, sondern auch quantitative Messungen möglich. Beispielhaft sei erwähnt, daß sich der Anteil an - noch unvernetzten - C=C-Doppelbindungen der eingesetzten Harzkomponenten, welche repräsentativ für die Viskosität der Duroplast-Zusammensetzung sind, mittels FIR bestimmen läßt.

Ein weiteres bevorzugtes spektroskopisches Verfahren auf der Basis von elektromagnetischer Strahlung stellt ein solches auf der Basis von ultravioletter Strahlung und/oder sichtbarem Licht, insbesondere UV/VIS, dar. Auch hier gilt im wesentlichen das zu IR-Spektren gesagte, wobei aufgrund der Absorptionsbanden in den erhaltenen UV/VIS-Spektren auf vorhandene Moleküle oder Molekülgruppen geschlossen werden, welche wiederum repräsentativ für die Viskosität der reifenden Duroplast-Zusammensetzung sind und - nach der weiter oben erwähnten Korrelation mit den direkt bzw. nicht zerstörungsfrei ermittelten Viskositätswerten - eine unmittelbare Ermittlung der Viskosität bzw. des Reifungszustandes der Duroplast-Zusammensetzung erlauben.

Eine andere bevorzugte Ausführungsform des erfindungsgemäßen Verfahrens sieht alternativ oder zusätzlich vor, daß ein spektroskopisches Verfahren auf der Basis von magnetischen und/oder elektromagnetischen Feldern, insbesondere Kernresonanzspektroskopie (Nuclear Magnetic Resonance, NMR), eingesetzt wird. Es können in diesem Fall z.B. Probenköpfe mit einem oder mehreren, z.B. zwei, Permanentmagneten und einer Oberflächenspule zum Einsatz kommen, wobei der Probenkopf im Oberflächenbereich der reifenden Duroplast-Zusammensetzung angeordnet ist. Die erhaltenen NMR-Spektren lassen ebenso wie (i) Relaxationszeiten (T₂ und/oder T₁) sowie Anteile von Phasen und (ii) mittlere Relaxationszeiten ebenfalls auf vorhandene Moleküle oder Molekülgruppen schließen, welche wiederum repräsentativ für die Viskosität der reifenden Duroplast-Zusammensetzung sind und - nach der weiter oben erwähnten Korrelation mit den direkt bzw. nicht zerstörungsfrei ermittelten Viskositätswerten - eine unmittelbare Ermittlung der Viskosität bzw. des Reifungszustandes der Duroplast-Zusammensetzung erlauben. Grundsätzlich kann zu diesem Zweck sowohl hoch- als auch niedrig aufgelöste NMR eingesetzt werden.

Gemäß einer Ausführungsform kann vorgesehen sein, daß elektromagnetische Strahlung bzw. magnetische Felder in die reifende Duroplast-Zusammensetzung eingeleitet werden, deren Frequenz der Absorptions- bzw. Resonanzfrequenz wenigstens einer Materialkomponente der Duroplast-Zusammensetzung und/oder Di- und/oder Oligomeren derselben entspricht. Bei der Materialkomponente kann es sich beispielsweise um eine der eingesetzten Komponenten, beispielsweise eine Harzkomponente, um während der Vernetzung gebildete kleine Moleküle, wie Wasser etc., oder auch um andere in der Duroplast-Zusammensetzung gegebenenfalls vorhandene Substanzen, wie Magnesiumoxid usw., handeln. Vorzugsweise werden dabei mehrere Spektralpunkte bzw. Spektralbereiche zur Erstellung der Korrelation mit den mittels der herkömmlichen, nicht zerstörungsfreien Verfahren ermittelten Viskositätswerten herangezogen. Indes kann grundsätzlich auch ein beliebiger, insbesondere relativ breiter Spektralbereich zur Ermittlung dieser Korrelation herangezogen werden und müssen hierzu nicht notwendigerweise diskrete Spektren von bekannten (Ausgangs)komponenten der Duroplast-Zusammensetzung verwendet werden.

Wie bereits angedeutet, werden im Falle des Einsatzes eines zerstörungsfreien Meßverfahrens auf der Basis von Ultraschall vorzugsweise die gemessene Schallgeschwindigkeit und/oder die gemessenen Dämpfungs- bzw. Absorptionseigenschaften der reifenden Duroplast-Zusammensetzung zur Ermittlung dieser Korrelation herangezogen.

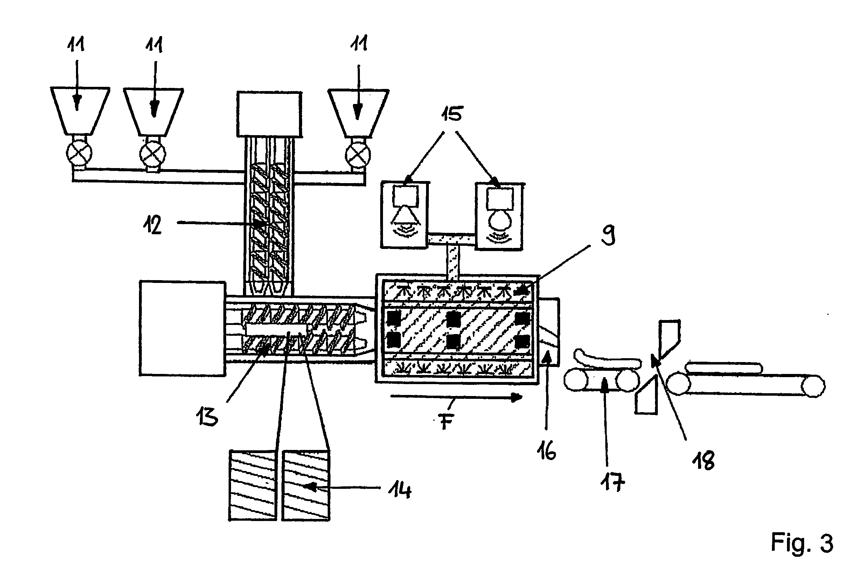

Eine besonders vorteilhafte Ausgestaltung des erfindungsgemäßen Verfahrens sieht vor, daß die spektroskopische Messung oder die Ultraschallmessung der Duroplast-Zusammensetzung ausschließlich an deren Oberfläche, insbesondere berührungsfrei, durchgeführt wird. Bei einer gattungsgemäßen Vorrichtung zur Herstellung duroplastischer Halbzeuge ist in diesem Fall folglich vorgesehen, daß der jeweilige Sensor und/oder das Ultraschallmikrofon ausschließlich, insbesondere berührungsfrei, mit der Oberfläche der in der Reifungseinheit befindlichen duroplastischen Halbzeuge zusammenwirkt. Eine solche, in "Aufsetztechnik" auf die Oberfläche des reifenden Duroplast-Halbzeugs durchgeführte Messung ist gänzlich zerstörungsfrei und nicht invasiv, so daß sie sich sowohl in Verbindung mit dem eingangs erwähnten Fließpreßverfahren, als auch im Falle des Einsatzes eines insbesondere weiter unten unter Bezugnahme auf Fig. 3 erläuterten Extrusionsverfahren arbeitet. Basiert die zerstörungsfrei Messung z.B. auf NMR, so lassen sich bereits bekannte NMR-Handgeräte, welche die NMR-Relaxation und -Diffusionsparameter in oberflächennahen Matrixbereichen messen, sowohl in Fließpreßmaschinen als auch in Extruder integrieren. Auf diese Weise ist eine "online"- bzw. Echtzeitbeurteilung der Duroplastmasse während der Reifung durchführbar.

Die spektroskopischen Messungen sowie die Ultraschallmessungen können im übrigen in Transmission durch die und/oder in Reflexion an der reifende(n) Duroplast-Zusammensetzung durchgeführt werden, d.h. Sender und Sensor werden entweder an ein und derselben Seite oder am entgegengesetzten Seiten der reifenden Duroplast-Zusammensetzung angeordnet.

Schließlich eröffnet das erfindungsgemäße Verfahren insbesondere die Möglichkeit, daß die Ermittlung der Viskosität der Duroplast-Zusammensetzung ausschließlich mittels der - zerstörungsfreien - spektroskopischen Verfahren oder Ultraschall unter Berücksichtigung der zuvor erhaltenen Korrelation kontinuierlich oder semikontinuierlich durchgeführt wird.

Nachstehend ist die Erfindung anhand von Ausführungsbeispielen unter Bezugnahme auf die Zeichnungen näher erläutert. In der Zeichnung zeigen:
- Fig. 1: ein Schaubild des Reifungsverhaltens einschließlich des nachfolgenden Aushärtens einer typischen Duroplast-Zusammensetzung, wobei die Viskosität η der Duroplast-Zusammensetzung als Funktion der Zeit t in doppelt logarithmischer Skalierung wiedergegeben ist (siehe hierzu weiter oben);
- Fig. 2: eine schematische perspektivische Ansicht einer Ausführungsform einer Vorrichtung zur Herstellung von duroplastischen Halbzeugen mit einer Reifungseinheit, welche eine zerstörungsfreie Echtzeitmessung der Viskosität der Halbzeuge ermöglicht; und
- Fig. 3: eine schematische, teilweise geschnitten dargestellte Seitenansicht einer anderen Ausführungsform einer Vorrichtung zur Herstellung von duroplastischen Halbzeugen mit einer Reifungseinheit, welche eine zerstörungsfreie Echtzeitmessung der Viskosität der Halbzeuge ermöglicht.

Bei der in Fig. 2 wiedergegebenen Vorrichtung, welche nach dem bekannten Fließpreßverfahren arbeitet, werden Verstärkungsfasern 1 von Spulen (nicht dargestellt) abgezogen und mittels einer Schneideinrichtung 2 auf die jeweils gewünschte Länge, z.B. etwa 25 mm, zugeschnitten. Die geschnittenen Fasern 3 fallen auf eine erste Trägerfolie 4, auf welche ein Anteil, z.B. etwa die Hälfte, der gewünschten Menge an Harz-/Füllstoffgemisch 5 in Form eines dünnen Filmes aufgebracht worden ist. Das Harz-/Füllstoffgemisch 5 ist zuvor in einer nicht wiedergegebenen Mischeinrichtung durch Homogenisieren der eingesetzten Komponenten erzeugt worden. Nach Abdecken dieser ersten Trägerfolie 4 mittels einer zweiten Trägerfolie 6, welche mit dem restlichen Anteil der gewünschten Menge an Harz-/Füllstoffgemisch 5 beschichtet worden ist, passiert die solchermaßen erzeugte sandwichartige Folienanordnung 4, 5, 6 eine Walzenstrecke mit einer Mehrzahl an beidseitig der Folienanordnung 4, 5, 6 positionierten Druckwalzen 7, so daß eine einwandfreie Imprägnierung der Fasern 3 mit dem Harz-/Füllstoffgemisch 5 erreicht und das gebildete Halbzeug 8 verdichtet wird. Das flächige Halbzeug 8 kann sodann z.B. aufgewickelt oder zugeschnitten werden.

Während grundsätzlich vorgesehen sein kann, daß eine Reifung der Halbzeuge 8 anläßlich einer anschließenden Lagerung der Halbzeuge 8 über mehrere Tage bis Wochen geschieht, ist erfindungsgemäß vorgesehen, daß die Vorrichtung mit einer - strichliniert dargestellten - Reifungseinheit 9 ausgestattet ist, welche z.B. unmittelbar stromab der Druckwalzen 7 angeordnet ist. Die Reifungseinheit 9 sorgt für einen hinreichenden Energieeintrag in das Halbzeug 8, um eine mit einer Erhöhung seiner Viskosität verbundene, beschleunigte Reifung bzw. Vorvernetzung der Duroplast-Zusammensetzung zu erzielen, während das Halbzeug 8 an der Reifungseinheit 9 vorbei geführt wird. Zur Energiezufuhr an das Halbzeug 8 kann die Reifungseinheit 9 z.B. mit in Fig. 2 nicht näher gezeigten Heizstrahlern (IR-Strahlern) oder andersartigen Bestrahlungseinrichtungen, z.B. UV-Strahlern oder Mikrowellenstrahlern oder auch radioaktiven Strahlungsquellen, wie α-, β- oder γ-Strahlern, ausgestattet sein, welche für einen für die gewünschte Reifung bzw. Vorvernetzung hinreichenden Energieeintrag sorgen.

Wiederum stromab der Reifungseinheit 9 passiert das Halbzeug 8 eine Mehrzahl an Meßeinheiten 10, welche zur zerstörungsfreien Echtzeitmessung der Viskosität des gereiften Halbzeugs 8 ausgebildet sind. Die Meßeinheiten 10 erstrekken sich beispielsweise über die gesamte Breite des Halbzeugs 8 und umfassen insbesondere jeweils wenigstens eine elektromagnetische Strahlungsquelle sowie einen zugehörigen Sensor zum Erfassen der jeweiligen elektromagnetischen Strahlungsspektren (z.B. auf der Basis von IR oder UV/VIS) und/oder wenigstens einen Elektromagnet sowie einen NMR-Sensor zum Erfassen der jeweiligen Kernresonanzspektren und/oder wenigstens einen Ultraschallsender sowie ein Ultraschallmikrofon zum Erfassen der jeweiligen Ultraschallsignale. Die Meßeinheiten 10 sind dabei insbesondere bezüglich der Duroplast-Anordnung 4, 5, 6 berührungsfrei angeordnet. Die Sensoren bzw. Ultraschallmikrofone der Meßeinheiten 10 sind an eine nicht gezeigte Auswerteeinheit angeschlossen, welche die erhaltenen Meßwerte anhand einer zuvor unter Heranziehen von experimentell bestimmten Viskositätswerten derselben Duroplast-Zusammensetzung ermittelten Korrelation auswertet und auf diese Weise die aktuellen Viskositätswerte ausgibt. Zusätzlich können z.B., sofern gewünscht, auch im Bereich der Reifungseinheit 9 weitere Meßeinheiten vorgesehen sein. Die insbesondere kontinuierliche Ermittlung des Reifezustandes bzw. der Viskosität der Halbzeuge 8 stellt eine wirksame Qualitäts- und Prozeßkontrolle unter weitestgehender Vermeidung von Ausschuß und Ausfallzeiten der Vorrichtung dar.

Die in Fig. 3 wiedergegebene Vorrichtung umfaßt eine Mehrzahl an Dosiereinrichtungen 11, über welche die Ausgangskomponenten einer jeweiligen Duroplast-Zusammensetzung, wie Harze, Füllstoffe und Additive, einer Mischeinrichtung in Form eines ersten Extruders 12, hier: eines Doppelschneckenextruders, aufgegeben werden. Die in dem ersten Extruder 12 vorgemischte Duroplast-Zusammensetzung gelangt von dort in einen zweiten Extruder 13, welcher beim vorliegenden Ausführungsbeispiel ebenfalls in Form eines Doppelschneckenextruders ausgebildet ist und in welchen auf Spulen 14 aufgewickelte Verstärkungsfasern, z.B. Glasfasern, eingezogen werden. Im Innern des zweiten Extruders 13 werden die Fasern zerteilt und mit der Duroplast-Zusammensetzung in innigen Kontakt gebracht.

An die Austrittsöffnung des zweiten Extruders 13 schließt sich eine Reifungseinheit 9 an, in welcher die Duroplast-zusammensetzung (Harz-/Füllstoffgemisch) - durch Energieeintrag beschleunigt - einem Reifungsprozeß unterworfen wird, anläßlich dessen eine Teilvernetzung oder Mikrogelierung ("micro-gelation", d.h. eine Vernetzung in einem räumlich begrenzten Bereich unter Erhöhung der Molmasse) der eingesetzten Harze stattfindet, womit eine Erhöhung der Viskosität der Duroplast-Zusammensetzung einhergeht (vgl. Fig. 1). Der Energieeintrag kann wiederum insbesondere in Form von Wärme und/oder Strahlung erfolgen, wobei im vorliegenden Fall UV- oder Mikrowellenstrahlung vorgesehen ist, welche aus einer jeweiligen Strahlungsquelle 15 in die Reifungseinheit 9 eingekoppelt wird. Die Reifungseinheit 9 ist erfindungsgemäß entsprechend der Reifungseinheit der in Fig. 1 wiedergegebenen Vorrichtung mit einer Mehrzahl an Meßeinheiten 10 ausgestattet, welche zur zerstörungsfreien Echtzeitmessung der Viskosität der reifenden Duroplast-Zusammensetzung ausgebildet sind. Die Meßeinheiten 10 sind beispielsweise sowohl in Längs- als auch in Querreihen bezüglich der mittels des Pfeils F angedeuteten Förderrichtung durch die Reifungseinheit 9 angeordnet, um den Reifungsvorgang bzw. die hiermit verbundene Viskositätserhöhung der Duroplast-Zusammensetzung zu erfassen. Sie können beispielsweise jeweils wenigstens eine elektromagnetische Strahlungsquelle sowie einen zugehöriger Sensor zum Erfassen der jeweiligen elektromagnetischen Strahlungsspektren (z.B. auf der Basis von IR oder UV/VIS), wenigstens einen Elektromagnet sowie einen NMR-Sensor zum Erfassen der jeweiligen Kernresonanzspektren (und/oder der jeweiligen Relaxationszeiten und Phasenanteilen bzw. mittleren Relaxationszeiten) und/oder wenigstens einen Ultraschallsender sowie ein Ultraschallmikrofon zum Erfassen der jeweiligen Ultraschallsignale aufweisen. Die Sensoren bzw. Ultraschallmikrofone der Meßeinheiten 10 sind an eine Auswerteeinheit (nicht dargestellt) angeschlossen, welche die erhaltenen Meßwerte anhand einer zuvor unter Heranziehen von experimentell bestimmten Viskositätswerten derselben Duroplast-Zusammensetzung ermittelten Korrelation auswertet und auf diese Weise die aktuellen Viskositätswerte ausgibt.

Aus der Reifungseinheit 9 wird die gereifte Duroplast-Zusammensetzung über eine Flachdüse 16 auf einen Bandförderer 17 überführt, welchem eine Schneideinrichtung 18 zum Ablängen der erzeugten Halbzeuge in den gewünschten Abmessungen zugeordnet ist.

## Patentansprüche

1. Verfahren zur Ermittlung des Reifezustandes, insbesondere der Viskosität, von Duroplast-Zusammensetzungen, **dadurch gekennzeichnet, daß** die Viskosität einer jeweiligen Duroplast-Zusammensetzung zerstörungsfrei ermittelt wird, indem zunächst zu verschiedenen Zeitpunkten während des Reifens der Duroplast-Zusammensetzung die Viskosität wenigstens einer Probe dieser Zusammensetzung mittels herkömmlicher, nicht zerstörungsfreier Verfahren zur Ermittlung der Viskosität bestimmt wird, wobei die Duroplast-Zusammensetzung im wesentlichen zu denselben Zeitpunkten mittels spektroskopischer Verfahren oder Ultraschall untersucht wird, wonach aus den erhaltenen Meßwerten eine Korrelation der mittels der spektroskopischen Verfahren oder Ultraschall erhaltenen Meßwerte mit den mittels der herkömmlichen Verfahren erhaltenen Viskositätswerte erstellt wird, und indem anschließend die Viskosität dieser Duroplast-Zusammensetzung ausschließlich mittels der spektroskopischen Verfahren oder Ultraschall unter Berücksichtigung der erhaltenen Korrelation ermittelt wird.

2. Verfahren nach Anspruch 1, **dadurch gekennzeichnet, daß** ein spektroskopisches Verfahren auf der Basis von elektromagnetischer Strahlung eingesetzt wird.

3. Verfahren nach Anspruch 2, **dadurch gekennzeichnet, daß** ein spektroskopisches Verfahren auf der Basis von Infrarot-Strahlung, insbesondere nahe Infrarot-Strahlung (NIR), mittlere Infrarot-Strahlung (MIR) und/oder ferne Infrarot-Strahlung (FIR), eingesetzt wird.

4. Verfahren nach Anspruch 2, **dadurch gekennzeichnet, daß** ein spektroskopisches Verfahren auf der Basis von ultravioletter Strahlung und/oder sichtbarem Licht, insbesondere UV/VIS, eingesetzt wird.

5. Verfahren nach Anspruch 2, **dadurch gekennzeichnet, daß** ein spektroskopisches Verfahren auf der Basis von magnetischen und/oder elektromagnetischen Feldern, insbesondere Kernresonanzspektroskopie (Nuclear Magnetic Resonance, NMR), eingesetzt wird.

6. Verfahren nach einem der Ansprüche 1 bis 5, **dadurch gekennzeichnet, daß** elektromagnetische Strahlung bzw. magnetische Felder in die reifende Duroplast-Zusammensetzung eingeleitet werden, deren Frequenz der Absorptions- bzw. Resonanzfrequenz wenigstens einer Materialkomponente der Duroplast-Zusammensetzung und/oder Di- und/oder Oligomeren derselben entspricht.

7. Verfahren nach einem der Ansprüche 1 bis 6, **dadurch gekennzeichnet, daß** die spektroskopische Messung oder die Ultraschallmessung der Duroplast-Zusammensetzung ausschließlich an deren Oberfläche, insbesondere berührungsfrei, durchgeführt wird.

8. Verfahren nach einem der Ansprüche 1 bis 7, **dadurch gekennzeichnet, daß** die spektroskopischen - oder Ultraschallmessungen in Transmission durch die und/oder in Reflexion an der reifende(n) Duroplast-Zusammensetzung durchgeführt werden.

9. Verfahren nach einem der Ansprüche 1 bis 8, **dadurch gekennzeichnet, daß** die Ermittlung der Viskosität der Duroplast-Zusammensetzung ausschließlich mittels der spektroskopischen Verfahren oder Ultraschall unter Berücksichtigung der zuvor erhaltenen Korrelation kontinuierlich oder semikontinuierlich durchgeführt wird.

10. Vorrichtung zur Herstellung duroplastischer Halbzeuge mit einer Mischeinrichtung zur Homogenisierung der eingesetzten Komponenten und einer Reifungseinheit (9), insbesondere in Form einer Bestrahlungs- und/oder Scherungseinheit, insbesondere zur Durchführung eines Verfahrens nach einem der Ansprüche 1 bis 9, **dadurch gekennzeichnet, daß** der Reifungseinheit (9)
- wenigstens eine zerstörungsfreie Meßeinheit (10) in Form einer elektromagnetischen Strahlungsquelle sowie eines zugehörigen Sensors zum Erfassen der jeweiligen elektromagnetischen Strahlungsspektren; und/oder
- wenigstens eines Elektromagnets sowie eines NMR-Sensors zum Erfassen der jeweiligen Kernresonanzspektren; und/oder
- wenigstens eines Ultraschallsenders sowie eines Ultraschallmikrofons zum Erfassen der jeweiligen Ultraschallsignale zugeordnet ist, wobei der jeweilige Sensor und/oder das Ultraschallmikrofon mit einer Auswerteeinheit korrespondiert.

11. Vorrichtung nach Anspruch 10, **dadurch gekennzeichnet, daß** der jeweilige Sensor und/oder das Ultraschallmikrofon ausschließlich, insbesondere berührungsfrei, mit der Oberfläche der in der Reifungseinheit befindlichen duroplastischen Halbzeuge zusammenwirkt.
